# EUROPEAN PATENT SPECIFICATION

(11) **EP 3 946 276 B1**
(45) Date of publication and mention of the grant of the patent: **13.12.2023**
(21) Application number: 20721146.7
(22) Date of filing: 23.03.2020
(51) Int. Cl.: A61K 9/20, A61K 31/137, A61K 9/14, A61K 9/16

(54) **SUSTAINED RELEASE COMPOSITION COMPRISING TAPENTADOL AND METHOD OF PREPARATION THEREOF**
ZUSAMMENSETZUNG MIT VERZÖGERTER FREISETZUNG MIT TAPENTADOL UND VERFAHREN ZUR HERSTELLUNG DAVON
COMPOSITION À LIBÉRATION PROLONGÉE COMPRENANT DU TAPENTADOL ET SON PROCÉDÉ DE PRÉPARATION

(30) Priority: 26.03.2019 GR 20190100148
(43) Date of publication of application: 09.02.2022
(73) Proprietor: Pharmathen S.A., 15351 Pallini Attikis (GR)
(72) Inventor: KARAVAS, Evangelos, 153 51 Pallini Attikis (GR); KOUTRIS, Efthymios, 153 51 Pallini Attikis (GR); SAMARA, Vasiliki, 153 51 Pallini Attikis (GR); KOUTRI, Ioanna, 153 51 Pallini Attikis (GR); KALASKANI, Anastasia, 153 51 Pallini Attikis (GR); KAKOURIS, Andreas, 153 51 Pallini Attikis (GR); SHAH, Rumit Rajivbhai, 153 51 Pallini Attikis (GR)
(86) International application number: PCT/EP2020/025140
(87) International publication number: WO 2020/192970

(56) References cited:
- WO-A1-2017/070462
- WO-A1-2017/070566
- US-A1- 2012 015 031

## Description

### TECHNICAL FIELD OF INVENTION

The present invention relates to a sustained release and abuse proof composition comprising Tapentadol or a pharmaceutically acceptable salt thereof for oral administration for the treatment of severe chronic pain in adults. Furthermore, the present invention describes a method of preparation of such pharmaceutical composition that is cost effective and will also increase patient compliance.

### BACKGROUND OF THE INVENTION

Tapentadol is well known as a centrally acting opioid analgesic of the benzoid class. It has a dual mode of action as an agonist of the µ-opioid receptor and as a norepinephrine reuptake inhibitor. Compared to morphine it shows 18-times reduced affinity to recombinant µ-opioid receptor suggesting that other pathways may contribute to its analgesic efficacy. Tapentadol was first described in EP 0693475.

In general, the release kinetics of the pharmacologically active ingredients is an important factor. It is well known that depending on how a pharmacologically active ingredient is formulated into a dosage form its release pattern can be modified. Controlled release also known as delayed release, prolonged release, sustained release, extended release and the like, may be based upon various concepts such as coating the pharmaceutical dosage form with a controlled release membrane, embedding the pharmacologically active ingredient in a matrix, binding the pharmacologically active ingredient to an ion-exchange resin, forming a complex of the pharmacologically active ingredient, and the like.

In comparison to formulations providing immediate release, formulations providing prolonged release upon oral administration have the advantage that they need to be administered less frequently, typically once daily or twice daily. This can reduce the peak plasma concentration of the pharmacologically active ingredient and any fluctuations that might occur with immediate release compositions which in turn may improve tolerability and increase patient compliance.

Many active pharmaceutical ingredients, besides having an excellent therapeutic activity in their suitable therapeutic prescription, also have a potential for abuse, i.e. can be used inappropriately by a user to achieve different effects from those expected. Opiates, for example, which are highly active in the treatment of severe or very severe pain, are often used by drug addicts to induce a state of narcosis or euphoria.

However, in order to abuse the active ingredient, the user pulverizes the corresponding pharmaceutical compositions, such as through grinding, and extracts the active ingredient from the powder thus obtained with the aid of a liquid, usually water, preferably in the minimum amount required to use in a hypodermic syringe. The resulting solution, possibly after filtration through cellulose wadding or cotton, is administered parenterally, particularly intravenously.

To prevent abuse of drugs containing substances with potential for abuse, some authorities such as the FDA (Food and Drug Administration), issued directives with the aim of assisting companies in developing medicines that have features that exert a deterrent action such as to discourage abuse and misuse.

Techniques known in the art to discourage the abuse can be grouped into five categories depending on the approach used. A first approach is to add a gelling or thickening agent to the pharmaceutical composition, making it impossible to attempt to abuse the formulation by solubilization in water. Alternately, a material with high mechanical strength is used that makes it impossible to attempt to abuse the formulation by grinding and inhaling. A third way is to introduce an agent able to antagonize and void the effect of the active ingredient comprised in the formulation if administered in different ways than expected. In addition, a sort of physical protection of the active ingredient is also made through a coating layer of hydrophobic substances which makes impossible the solubilization. One last technique involves the addition of pollutants, for example irritants, which make taking the formulation by inhalation and/or injection unpleasant.

WO2013/030177 relates to an abuse-proof tablet including an active layer comprising oxycodone and acetaminophen and a gelling layer comprising hydroxypropyl methylcellulose.

WO95/20947 relates to an abuse-proof tablet comprising two or more layers, where one or more substances with potential for abuse and one or more gelling substances are contained in separate layers. The gelling substances included modified cellulose, sodium alginate, alginic acid, polyacrylic acid, tragacanth, xanthan gum, guar gum, and carob flour.

WO2008/150526 relates to a delivery system consisting of a lipid solid suspension comprising, in addition to an active substance, at least one gelling agent and a lipid in a weight ratio of less than 1: 1.4.

WO2014/123899 describes a solid dosage form, in particular a tablet, containing a heat-labile gelling agent, in particular xanthan gum, a heat stabilizer, in particular carbomer (crosslinked homopolymers of acrylic acid) and a substance with potential for abuse.

WO 2007/093642 claims a modified release form aiming at minimizing the risks of release of the dose associated with the concurrent consumption of alcohol and certain pharmaceutical or dietary forms.

However, there still exists the need to develop alternative oral formulations that are abuse proof which provide medicinal action while at the same time having the benefits of controlled or modified release formulations.

### SUMMARY OF THE INVENTION

It is, therefore, an object of the present invention to provide a pharmaceutical formulation comprising Tapentadol or a pharmaceutically acceptable salt thereof as set out in the appended claims, that has good physicochemical properties. The composition of the present invention is a sustained release composition with a drug release profile of more than 80% after 12 hours of administration.

Another object of the present invention is the preparation of pharmaceutical composition comprising Tapentadol or pharmaceutically acceptable salt thereof together with an agent or a combination of agents for sustained release of the active ingredient.

Furthermore, an object of the present invention is to have a composition that is abuse proof and shows a combination of abuse proof qualities such as tablet hardness and increased viscosity. The tablet described in the preset invention has a hardness of above 500N and an increased viscosity when diluted in water. The dosage form of the present invention is therefore suitable for preventing parenteral, nasal and oral abuse of the active ingredient.

A further object of the present invention is to provide a sustained release and abuse proof pharmaceutical formulation comprising Tapentadol or a pharmaceutically acceptable salt thereof that has a sustainable manufacturing process. The manufacturing process of the present invention comprising hot melt extrusion of the active ingredient together with hydrophilic and hydrophobic polymer(s) and optionally adding viscosity enhancing agents.

### DETAILED DESCRIPTION OF THE INVENTION

As used herein, "sustained release" means that the release of the active ingredient is substantially slower than immediate release. Examples of such sustained release include controlled release, slow release, prolonged release, delayed release, pulsatile release, extended release, timed release etc., which terms are generally known in the art and to the extent they mean a release other than an immediate release. Controlled release may be achieved by various technologies such as reservoir, matrix, osmotic, gastro-retention, bioadhesion, complexation, conjugation etc.

For the purpose of the present invention Tapentadol includes various forms of Tapentadol such as pharmaceutically acceptable salt(s), hydrate(s), solvate(s), polymorph(s), isomer(s), stereoisomer(s), enantiomer(s), racemate(s), ester(s), prodrug(s), derivative(s), analogou(s), metabolite(s) and complex(s) thereof. More specifically for the present invention the preferred active ingredient is Tapentadol and it may be present from 1 to 90% w/w of the total composition.

The term "pharmaceutically acceptable salt" means a salt which is acceptable for administration to a patient, such as a mammal (e.g., salts having acceptable mammalian safety for a given dosage regime). Such salts can be derived from pharmaceutically acceptable inorganic or organic bases and from pharmaceutically acceptable inorganic or organic acids.

Composition includes, without limitation, tablets, capsules, caplets, powders, pellets, granules, liquid dispersions, beads, etc. Powders, pellets, and granules may be coated with a suitable polymer or a conventional coating material to achieve, for example, greater stability in the gastrointestinal tract, or to achieve the desired rate of release. Tablets may be minitablets, multi-layered tablets, coated or uncoated tablets, tablet in tablet etc. those are known in art. It may also include kits. These compositions can be administered orally.

The term "core" as used herein may be a part of the composition surrounded by at least a part of the coating or layer. The core can be homogenous or have an internal structure comprising powder, particles, granules, pellets, tablets, minitablets, capsules, caplets, or a mixture thereof, comprising active ingredient(s) or carriers/ substrates or a mixture thereof. Core may be prepared by addition of excipients, binder, disintegrant, lubricant and so on, as would be understood by one of ordinary skill in the art.

As used herein, "%" refers to the weight percent of a substance as it relates to the overall composition unless otherwise indicated.

The term "comprising", which is synonymous with "including", "containing", or "characterized by" here is defined as being inclusive or open-ended, and does not exclude additional, unrecited elements or method steps, unless the context clearly requires otherwise.

The once daily sustained release pharmaceutical composition comprises Tapentadol and one or more pharmaceutically acceptable excipient(s) which includes release controlling agents, and may optionally contain binders, diluents, lubricants, glidants and plasticizers etc. The amount of excipient employed will depend upon the quantity of active ingredient to be used.

Therefore, the main objective of the present invention is the preparation of a sustained release pharmaceutical composition comprising Tapentadol or a pharmaceutically acceptable salt that is also abuse proof.

Agents for sustained release are defined as hydrophilic or hydrophobic agents, which can be polymeric or non-polymeric and which are capable of controlling the rate or release of the active agent(s).

The hydrophobic polymers for sustained controlled release of active ingredients are the release controlling agents which delay the release of the active ingredients from the composition. Non limiting examples of this type of polymers include cellulosic derivatives including ethylcellulose, hydroxypropyl cellulose, hydroxypropyl methyl cellulose, hydroxyethyl cellulose, sodium carboxy methylcellulose, calcium carboxymethyl cellulose, methyl cellulose, as well as noncellulosics such as maltodextrin, polyvinyls, polyvinyl alcohol, and co-polymers of acrylic and methacrylic acid esters (Eudragit^{®} RS or RL).

Ethylene vinyl acetate (EVA) excipients are also hydrophobic excipients which are used for controlled-release of drugs and medical implants and may be used alone or in combination with other excipients and/or additives to achieve the desired release-rate profile

Kollidon SR is a polyvinyl acetate/povidone based matrix retarding agent. It is particularly suitable for the manufacture of sustained release matrix tablets. Polyvinyl acetate is a plastic material that produces a coherent matrix even under low compression forces. The blend of polyvinyl acetate and povidone (K 30) used according to the present invention is in the ratio 8:2. It is a particularly preferred hydrophobic excipient because besides the ability of forming sustained release matrices, provides a higher breaking strength to dosage forms. In the present invention it is used in an amount of 15 to 30% w/w.

Hydrophilic polymers used as release controlling agents are those that that swell upon contact with water. Those can be selected from, but are not limited to, water-soluble polymers (hydrophilic) such as polyethylene oxide and cellulosic polymer derivatives including hydroxypropyl cellulose, hydroxypropyl methyl cellulose (HPMC), hydroxyethyl cellulose, sodium carboxy methylcellulose, calcium carboxymethyl cellulose, methyl cellulose, as well as noncellulosic such as maltodextrin, polyvinyls, polyvinyl alcohol, polyacrylic acids, alginates, gelatin, natural gums, including guar, lightly crosslinked versions of these polymers, starches, starch graft copolymers and the like. The polymers generally have number average molecular weights over 50,000 grams per mole, such as between 50,000 and 10,000,000 grams per mole. Polymers having molecular weights between 300,000 and 8,000 000 grams per mole are preferred, and those having molecular weights between about 2,000,000 to 8,000,000 grams per mole are especially preferred. Polyethylene oxide having a number average molecular weight between about 5,000,000 to 8,000,000 grams per mole is most especially preferred, e.g. Polyox 303 and Polyox 308. Also, especially preferred are methylcellulose type/grade A15C, A4M, A18 M and hydroxypropyl methylcellulose type/grade K4M, K15M, K100M, E4M and F4M (Dow Chemical Company); hydroxyethyl cellulose such as Natrosole HEC; hydroxypropyl cellulose such as Klucel (Grades H, M, G, J, L, E- Aqualon Company); guar such as Supercol^{®} Guar U (Aqualon Company); pectin such as GENU Pectin (Aqualon Company); carrageenan such as GENU Carrageenan (Aqualon Company); poly(methyl vinyl ether/maleic anhydride) such as Gantrez^{®} AN Copolymer (AN- 119, -139, -149, -169, -179, GAF Corporation); polyvinyl alcohol such as Elvanol^{®} 71-30, Elvanol^{®} 85-80, Elvanol^{®} 55- 65, Elvanol^{®} 50-42 and Elvanol^{®} HV (DuPont); sodium carboxymethyl cellulose such as Aqualon cellulose gum grade 7H4; polyacrylic acids such as Carpobol resin grades 934P, 940, 941, 971P, 974P, 980, 981, 1382, 2984, 5984, ETD 2001, ETD 2050, calcium polyacrylic acids such as Noveon^{®} resin grades AA-1, CA-1 and CA-2, and sodium polyacrylic acid (BF Goodrich, Cleveland, Ohio). More preferred is hydroxypropyl methylcellulose and combinations thereof.

The controlled release pharmaceutical composition of the present invention may also contain a surface-active agent or solubilizing agents. Solubilizing agents help to solubilize the active ingredient either in composition or in-situ at the site of absorption or action. Solubilizing agents include but are not limited to surfactants, cyclodextrin and its derivatives, lipophilic substances or any combination thereof. Non-limiting examples of surfactants include water soluble or water dispersible nonionic, semi-polar nonionic, anionic, cationic, amphoteric, or zwitterionic surface active agents or any combination thereof.

The preferred surface active agents include, but are not limited to, copolymers composed of a central hydrophobic chain of polyoxypropylene (poly (propylene oxide)) and polyoxyethylene (poly (ethylene oxide)) that is well known as poloxamer. However, other agents may also be employed such as dioctyl sodium sulfosuccinate (DSS), triethanolamine, sodium lauryl sulphate (SLS), polyoxyethylene sorbitan and poloxalkol derivatives, quaternary ammonium salts or other pharmaceutically acceptable surface active agents known to the person skilled in the art. Other solubilizing agents include but not necessarily limited to vitamin E and its derivatives; monohydric alcohol esters such as trialkyl citrates, lactones and lower alcohol fatty acid esters; nitrogen-containing solvents; phospholipids; glycerol acetates such as acetin, diacetin and triacetin; glycerol fatty acid esters such as mono-, di- and triglycerides and acetylated mono- and di-glycerides; propylene glycol esters; ethylene glycol esters and combinations thereof.

A particularly preferred excipient in the present invention is Kollidon^{®} VA 64. It is a vinylpyrrolidone-vinyl acetate copolymer and it is usually used as dry binder for direct compression tableting, as granulating agent, as retarding and as a film-forming agent as well as in taste-masking applications. However, it is ideal as a solubilizer in hot-melt extrusion processes.

The utilization of many polymer excipients in order to achieve the required hardness for abuse proof the hot melt extrusion manufacturing process is ideal. This technology offers several advantages over conventional pharmaceutical manufacturing processes such as shorter and more efficient time to achieve the final product, environmental advantages due to the elimination of solvent use, and increased efficiency of drug delivery to the patient. Other advantages include enhanced bioavailability, lower doses, and reduced production costs. Additional advantages include improved solubility, improved dispersion, improve stability, controlled release rates, volume and scale up flexibility, dose form and aesthetic flexibility.

Therefore, the present invention also provides a process for the production of the abuse-proofed dosage forms, which process is comprising:
A) All components are mixed together or separately in different combination and the resultant mixture or the resultant mixtures is/are heated in the extruder at least up to the softening point of Kollidon SR and extruded through the outlet orifice of the extruder by application of force,
B) the still plastic extrudate is cingulated and formed into the dosage form or the cooled and optionally reheated cingulated extrudate, is formed into the dosage form.

The process according to the invention is preferably performed using conventional extruders, particularly preferably screw extruders, which may be equipped with one or two screws. After heating at least up to the Softening point of Kollidon SR, the molten mixture is conveyed with the assistance of the screws and is s extruded through the die as an extruded Strand or strands. The die geometry is freely selectable. The die may exhibit a round or oblong cross-section.

Extruder chambers can be heated or cooled so as to achieve minimum softening temperature required and to avoid sudden rise of temperature at which the active substance with abuse potential may be damaged. Preferably, the temperature of the mixture to be extruded is adjusted to below 180°C., preferably below 150° C. After extrusion and optional cooling of the extruded strand or extruded strands a process of comminution performed by cutting up the extrudates by means of revolving or rotating knives, water jet cutters, wires, blades or with the assistance of laser cutters or any other means. Furthermore, after a process of comminution to impart final shape to the dosage form conventional or non-conventional methodologies can be applied such as coating. Polyethylene glycol is a particularly useful excipient in the manufacturing process of the present invention since it functions as a plasticizer and facilitates the hot melt extrusion process.

The dosage forms obtained by the production process according to the invention are distinguished in that, due to their hardness, they cannot be pulverised, for example by grinding in a mortar. This virtually rules out oral or parenteral, in particular intravenous or nasal abuse. However, in order to prevent any possible abuse of the dosage forms obtained by the production process according to the invention in the event of comminution and/or pulverisation which possibly occur nonetheless due to extraordinary force, in a preferred embodiment these dosage forms may contain further auxiliary substances.

An option for preventing abuse of the dosage form obtained by the process according to the invention consists in adding at least one viscosity-increasing agent as a further abuse-preventing component to the dosage form, which, with the assistance of a necessary minimum quantity of an aqueous liquid, forms a gel with the extract obtained from the dosage form, which gel is virtually impossible to administer safely and preferably remains visually distinguishable when introduced into a further quantity of an aqueous liquid.

For the purposes of the present invention, visually distinguishable means that the active ingredient-containing gel formed with the assistance of a necessary minimum quantity of aqueous liquid, when introduced, preferably with the assistance of a hypodermic needle, into a further quantity of aqueous liquid at 37°C., remains substantially insoluble and cohesive and cannot straightforwardly be dispersed in such a manner that it can safely be administered parenterally, in particular intravenously. The material preferably remains visually distinguishable for at least one minute, preferably for at least 10 minutes.

The increased viscosity of the extract makes it more difficult or even impossible for it to be passed through a needle or injected. If the gel remains visually distinguishable, this means that the gel obtained on introduction into a further quantity of aqueous liquid, for example by injection into blood, initially remains in the form of a largely cohesive thread, which, while it may indeed be broken up mechanically into smaller fragments, cannot be dispersed or even dissolved in such a manner that it can safely be administered parenterally, in particular intravenously. Intravenous administration of such a gel would therefore most probably result in serious damage to the health of the abuser.

One or more viscosity-increasing agents are used, which are selected from the group comprising microcrystalline cellulose, hydroxypropyl cellulose, hydroxypropylmethyl cellulose, hydroxyethyl cellulose, carboxymethylcellulose sodium, polyacrylic acid, locust bean flour, pectins, preferably from citrus fruits or apples, waxy maize starch, sodium alginate, guar flour, Polygum, iota-carrageenan, karaya gum, gellan gum, galactomannan, tara stone flour, propylene glycol alginate, sodium hyaluronate, tragacanth, tara gum, fermented polysaccharide welan gum, xanthans such as xanthan gum. Xanthans are particularly preferred. In general, a quantity of 0.1 to 5 wt. % of the viscosity-increasing agent(s) is enough to fulfil the above-stated conditions.

Viscosity increasing agents are preferably present in the dosage form obtained by the production process according to the invention in quantities of 0.1 to 25 % by weight, preferably of 5 to 15 % by weight, particularly preferably of 1-10 % weight of the total weight of the composition. The preferred viscosity agent for the present invention is hydroxyethyl cellulose and surprisingly its dual action as a viscosity enhancing agent and as a gelling and thickening agent greatly increase the dissolution profile of the final formulation to the desired range.

The composition of the present invention may also contain at least one binder. The binder used for this purpose is at least one polymer selected from among the group comprising polymethylene oxide, polyethylene oxide, polypropylene oxide, polyethylene, polypropylene, polyvinyl chloride, polycarbonate, polystyrene, polyacrylate, poly(hydroxyfatty acids), such as for example poly(3-hydroxybutyrate-co-3-hydroxyvalerate) (Biopol(R)), poly(hydroxyvaleric acid), polycaproplactone, polyvinyl alcohol, polyesteramides, polyethylene succinate, polylactones, polyglycolides, polyurethanes, polyamides, polylactides, polylactide/glycolide, polylactones, polyglycolides, polyorthoesters, polyanhydrides, block polymers of polyethylene glycol and polybutylene terephthalate (Polyactive(R)), polyanhydrides (Polifeprosan), the copolymers thereof, and mixtures of at least two of the stated polymers.

In order to achieve the necessary breaking strength with the production process according to the invention, it is furthermore possible additionally to use at least one natural or synthetic wax with a breaking strength, measured using the method disclosed in the present application, of at least 500 N. Waxes with a softening point of at least 60o C. are preferred. Carnauba wax and beeswax are particularly preferred. Carnauba wax is very particularly preferred. Carnauba wax is a natural wax which is obtained from the leaves of the carnauba palm and has a softening point of 80o C. When the wax component is additionally used, it is used together with at least one polymer in quantities such that the dosage form has a breaking strength of at least 500 N.

Fillers or diluents include, but are not limited to dextrates, dextrin, dextrose, fructose, lactitol, mannitol, sucrose, starch, lactose, xylitol, sorbitol, talc, microcrystalline cellulose, calcium carbonate, calcium phosphate dibasic or tribasic, calcium sulphate or mixtures thereof. The preferred filler or diluent amount may vary within the range of from about 10% to about 60% by weight of the total composition.

Lubricants may be selected from, but are not limited to, those conventionally known in the art such as magnesium, aluminium or calcium or zinc stearate, polyethylene glycol, glyceryl behenate, mineral oil, sodium stearyl fumarate, stearic acid, hydrogenated vegetable oil and talc or mixtures thereof. The preferred lubricant amount may vary within the range of from about 0.1% to about 2.0% by weight of the total composition and most preferably it is 1.0% by weight of the total composition.

Glidants include, but are not limited to, silicon dioxide, fumed silica, magnesium trisilicate, powdered cellulose, starch, talc and tribasic calcium phosphate, calcium silicate, magnesium silicate, colloidal silicon dioxide, silicon hydrogel or mixtures thereof. The prefer glidant amount may vary within the range of from about 0.1% to about 10% by weight of the total composition and most preferably it is 2% by weight of the total composition.

The once daily controlled release pharmaceutical composition may optionally have one or more non-functional coatings such as film coating or sugar coating, which has no or negligible impact on release of active ingredient form the composition. The controlled release pharmaceutical composition may further have one or more functional coating such as bioadhesive coating, diffusion coatings, non-permeable coating and semipermeable coating, which modify the release of active ingredients from the composition.

### EXAMPLES

### Example 1

The inventors performed initial trial formulations as shown in Table 1 below. The manufacturing process comprises weighing the excipients and the active ingredient and dry mixing until a pre-mix homogenous blend is achieved. Subjecting the pre-mix to hot melt extrusion at a temperature of below 180°C, in combination with a 8mm extrudate die. The extrudate is first cut into rods and then to tablets of oblong shape.

**Table 1: Quantitative and qualitative analysis of Formulation trials 1-10**

| **Formulation** | **1** | **2** | **3** | **4** | **5** |
|---|---|---|---|---|---|
| **Ingredients** | mg | | | | |
| **Tapentadol HCl** | 291.20 | 291.20 | 291.20 | 291.20 | 291.20 |
| **Kollidon SR** | 58.35 | 58.35 | 116.71 | 58.35 | 58.35 |
| **Kollidon VA64** | 58.35 | 58.35 | 116.71 | 224.75 | 242.08 |
| **EVA** | 242.08 | 224.75 | 116.71 | 58.35 | 58.35 |
| **HPMC K100M** | 21.67 | 30.33 | 26.00 | 30.33 | 21.67 |
| **PEG 6000** | 21.67 | 30.33 | 26.00 | 30.33 | 21.67 |
| **Total weight** | 693.32 | 693.32 | 693.33 | 693.32 | 693.32 |
| ***Hardness*** | *150 N* | *160 N* | *262 N* | *206 N* | *246 N* |

| **Formulation** | **6** | **7** | **8** | **9** | **10** |
|---|---|---|---|---|---|
| **Ingredients** | mg | | | | |
| **Tapentadol HCl** | 291.20 | 291.20 | 291.20 | 291.20 | 291.20 |
| **Kollidon SR** | - | - | 224.75 | 242.08 | 332.80 |
| **Kollidon VA64** | 332.80 | 367.47 | 58.35 | 58.35 | - |
| **EVA** | - | - | 58.35 | 58.35 | - |
| **HPMC K100M** | 34.67 | 17.33 | 30.33 | 21.67 | 34.67 |
| **PEG 6000** | 34.67 | 17.33 | 30.33 | 21.67 | 34.67 |
| **Total weight** | 693.33 | 693.33 | 693.32 | 693.32 | 693.33 |
| ***Hardness*** | *130 N* | *181 N* | *622 N* | *644 N* | *635 N* |

The results show that only Formulation trials 8-10 showed a breaking strength above the target 500N, however the dissolution profile for those formulations was not in the desired range.

### Example 2

In order to achieve the necessary breaking strength of at least 500N new formulation trials were developed and different combinations of EVA, Kollidon SR and HPMC were evaluated. The manufacturing process remained the same as in example 1. The Formulations tested are shown in Table 2.

**Table 2: Quantitative and qualitative analysis of Formulation trials 11-16**

| **Formulations** | **11** | **12** | **13** | **14** | **15** | **16** |
|---|---|---|---|---|---|---|
| **Ingredients** | mg | | | | | |
| **Tapentadol HCl** | 291.18 | 291.18 | 291.18 | 291.18 | 291.18 | 291.18 |
| **Kollidon SR** | 152.25 | 152.25 | 49.03 | 131.17 | 168.59 | 100.00 |
| **Kollidon VA64** | - | - | 29.91 | 48.88 | 39.78 | 140.00 |
| **HPMC K100M** | 49.59 | 49.59 | 34.66 | 33.62 | 34.32 | 50.00 |
| **PEG 6000** | 29.00 | 29.00 | 34.66 | 33.62 | 34.32 | 30.00 |
| **EVA 3325A** | 202.99 | - | - | - | - | - |
| **EVA 4030AC** | - | 202.99 | 253.83 | 154.81 | 125.09 | 84.00 |
| **Total Weight** | 725.01 | 725.01 | 693.28 | 693.28 | 693.28 | 695.18 |
| **Hardness** | 160N | 170N | 125N | 295N | 250N | 390N |

However, the results show that the hardness of at least 500N was still not reached.

### Example 3

Subsequently, different amounts of Kollidon SR and Kollidon VA64 to optimize tablet hardness were used. In addition, viscosity enhancing agents were added to impart additional abuse proof protection.

Different combinations of hydrophilic and hydrophobic polymers with viscosity increasing agents were studied. Kollidon SR was used as hydrophobic polymer in combination with one or more hydrophilic polymers that can be also be also categories as viscosity increasing agents. The formulation trials 17- 22 were prepared with the same manufacturing process as in example 1 and are shown below in Table 3.

**Table 3: Quantitative and qualitative analysis of Formulation trials 17-22**

| **Formulations** | **17** | **18** | **19** | **20** | **21** | **22** |
|---|---|---|---|---|---|---|
| **Ingredients** | mg | | | | | |
| **Tapentadol** | 291.18 | 291.18 | 291.18 | 291.18 | 291.18 | 291.18 |
| **Kollidon SR** | 180.00 | 180.00 | 180.00 | 150.00 | 110.00 | 130.00 |
| **Kollidon VA64** | 30.00 | 30.00 | 80.00 | 110.00 | 150.00 | 130.00 |
| **HPMC K100M** | 80.00 | 80.00 | 80.00 | 80.00 | 80.00 | 80.00 |
| **PEG 6000** | 30.00 | 30.00 | 30.00 | 30.00 | 30.00 | 30.00 |
| **Xanthan Gum** | 90 | - | - | - | - | - |
| **Guar Gum** | - | 90.00 | 40.00 | 40.00 | 40.00 | 40.00 |
| **Total Weight** | 701.18 | 701.18 | 701.18 | 701.18 | 701.18 | 701.18 |
| **Hardness** | 745N | 768N | 810N | 670N | 670N | 700N |

Formulations 17-22 were tested for viscosity and were comminuted and extracted with 10 ml of water at a temperature of 25° C. The increased viscosity shown for all formulations after extraction makes it more difficult or even impossible for any of them to be passed through a needle or injected. In addition, the hardness above 500 N match criteria for all. However, dissolution profile analysis measured as in example 1 showed that the dissolution percentage of formulations 17-19 was around 60-70% at 12 hours, whereas the dissolution percentage of formulations 20-22 was around 80-90% at 12 hours.

### Example 4

A further trial was performed using hydroxyethyl cellulose as a viscosity enhancer that can also act as a hydrophilic polymer. Formulations 23-29 are shown in Table 4 and the manufacturing process was the same as in example 1. Formulations 23-29 were tested for viscosity and were comminuted and extracted with 10 ml of water at a temperature of 25° C. The increased viscosity shown for all formulations after extraction makes it more difficult or even impossible for any of them to be passed through a needle or injected and the breaking strength was above 500N for all.

Dissolution profile analysis measured as in example 1 showed that the dissolution percentage of Formulations 23-27 was around 65-75% at 12 hours, whereas the dissolution percentage of Formulations 28 was around 80-90% at 12 hours and for Formulation 29 was around 90-95% at 12 hours. Surprisingly the addition of hydroxyethyl cellulose as a viscosity enhancer improves the dissolution profile of the formulation. Stability studies for Formulation 29 showed that the composition remains stable at 6 months under various conditions.

**Table 4: Quantitative and qualitative analysis of Formulation trials 23-29**

| **Formulations** | **23** | **24** | **25** | **26** | **27** | **28** | **29** |
|---|---|---|---|---|---|---|---|
| **Ingredients** | mg | | | | | | |
| **Tapentadol** | 291.18 | 291.18 | 291.18 | 291.18 | 291.18 | 291.18 | 291.18 |
| **Kollidon SR** | 250.00 | 200.00 | 180.00 | 150.00 | 180.00 | 150.00 | 150.00 |
| **Kollidon VA64** | - | - | 30.00 | 60.00 | 30.00 | 30.00 | 60.00 |
| **HPMC K100M** | 70.00 | 80.00 | 80.00 | 80.00 | 80.00 | 80.00 | 80.00 |
| **PEG 6000** | 30.00 | 30.00 | 30.00 | 30.00 | 30.00 | 30.00 | 30.00 |
| **Natrosol 250 HX** | 50.00 | 90.00 | 90.00 | 90.00 | 60.00 | 60.00 | 60.00 |
| **Total Weight** | 691.18 | 691.18 | 701.18 | 701.18 | 671.18 | 641.18 | 671.18 |
| **Hardness** | 626N | 750N | 820N | 600N | 790N | 720N | 815N |

## Claims

1. An abuse proof sustained release pharmaceutical composition for oral administration comprising Tapentadol or pharmaceutically acceptable salt thereof, a polyvinyl acetate/povidone blend of a ratio 8:2 as a hydrophobic polymer that imparts a breaking strength of at least 500N to said composition and hydroxypropylmethyl cellulose as a hydrophilic polymer, wherein the hydrophobic polymer is at a concentration of from 15% w/w to 30% w/w and the hydrophilic polymer from 10% w/w to 20% w/w.

2. The pharmaceutical composition according to claim 1, further comprising vinylpyrrolidone-vinyl acetate copolymer as a solubilizer.

3. The pharmaceutical composition according to claim 1, further comprising the viscosity enhancing agent hydroxyethyl cellulose.

4. A process for preparation of an abuse proof sustained release pharmaceutical composition for oral administration comprising Tapentadol or pharmaceutically acceptable salt thereof, a polyvinyl acetate/povidone blend of a ratio 8:2 as a hydrophobic polymer that imparts a breaking strength of at least 500N and hydroxypropylmethyl cellulose as a hydrophilic polymer, wherein the hydrophobic polymer is at a concentration of from 15% w/w to 30% w/w and the hydrophilic polymer from 10% w/w to 20% w/w, wherein said process is hot melt extrusion at a temperature of less than 180°C.

5. The process according to claim 4, wherein all components are mixed together or separately in different combination and the resultant mixture or the resultant mixtures is/are heated in the extruder at least up to the softening point of polyvinyl acetate/povidone blend and extruded through the outlet orifice of the extruder by application of force & the still plastic extrudate is cingulated and formed into the dosage form or the cooled and optionally reheated cingulated extrudate, is formed into the dosage form.

6. The process according to claim 4, wherein it further comprises a viscosity enhancing agent, a solubilizer and a plasticizer.

## Patentansprüche

1. Missbrauchssichere pharmazeutische Zusammensetzung mit verzögerter Wirkstofffreisetzung zur oralen Verabreichung, umfassend Tapentadol oder ein pharmazeutisch annehmbares Salz davon, eine Polyvinylacetat/Povidon-Mischung in einem Verhältnis von 8:2 als hydrophobes Polymer, das der Zusammensetzung eine Bruchfestigkeit von mindestens 500 N verleiht, und Hydroxypropylmethylcellulose als hydrophiles Polymer, **dadurch gekennzeichnet, dass** das hydrophobe Polymer in einem Gewichtprozent von 15% bis 30% und das hydrophile Polymer in einem Gewichtprozent von 10% bis 20% enthalten sind.

2. Pharmazeutische Zusammensetzung nach Anspruch 1, die ferner Vinylpyrrolidon-Vinylacetat-Copolymer als Lösungsvermittler umfasst.

3. Pharmazeutische Zusammensetzung nach Anspruch 1, die weiterhin das viskositätserhöhende Mittel Hydroxyethylcellulose enthält.

4. Verfahren für die Zubereitung einer missbrauchssicheren pharmazeutischen Zusammensetzung mit verzögerter Wirkstofffreisetzung zur oralen Verabreichung, umfassend Tapentadol oder ein pharmazeutisch verwendbares Salz davon, eine Polyvinylacetat/Povidon-Mischung in einem Verhältnis von 8:2 als hydrophobes Polymer, das eine Bruchfestigkeit von mindestens 500 N verleiht, und Hydroxypropylmethylcellulose als hydrophiles Polymer, **dadurch gekennzeichnet, dass** das hydrophobe Polymer in einem Gewichtprozent von 15% bis 30% und das hydrophile Polymer in einem Gewichtprozent von 10% bis 20% enthalten sind, wobei das Verfahren Heißschmelzextrusion bei einer Temperatur von weniger als 180 °C ist.

5. Verfahren nach Anspruch 4, **dadurch gekennzeichnet, dass** alle Komponenten zusammen oder getrennt in unterschiedlicher Kombination gemischt werden und die resultierende Mischung bzw. die resultierenden Mischungen im Extruder mindestens bis zum Erweichungspunkt der Polyvinylacetat/Povidon-Mischung erwärmt und unter Krafteinwirkung durch die Auslassöffnung des Extruders befördert wird bzw. werden und das noch plastische Extrudat zerkleinert und zur Darreichungsform geformt wird oder das abgekühlte und gegebenenfalls wieder erwärmte zerkleinerte Extrudat zur Darreichungsform geformt wird.

6. Verfahren nach Anspruch 4, **dadurch gekennzeichnet, dass** es ferner ein viskositätserhöhendes Mittel, einen Lösungsvermittler und ein Plastifiziermittel umfasst.

## Revendications

1. Composition pharmaceutique à libération prolongée permettant d'éviter les abus pour l'administration orale comprenant du tapentadol ou un sel pharmaceutiquement acceptable de celui-ci, un mélange d'acétate de polyvinyle/povidone dans un rapport de 8:2 en tant que polymère hydrophobe qui confère une résistance à la rupture d'au moins 500N à ladite composition et de l'hydroxypropyl méthylcellulose en tant que polymère hydrophile, dans laquelle le polymère hydrophobe est à une concentration de 15 % p/p à 30 % p/p et le polymère hydrophile de 10 % p/p à 20 % p/p.

2. Composition pharmaceutique selon la 1^{ère} revendication, comprenant en outre un copolymère vinyl pyrrolidone-acétate de vinyle comme solubilisant.

3. Composition pharmaceutique selon la 1^{ère} revendication, comprenant en outre l'agent hydroxyéthylcellulose qui améliore la viscosité.

4. Composition pharmaceutique à libération prolongée permettant d'éviter les abus pour l'administration orale comprenant du tapentadol ou un sel pharmaceutiquement acceptable de celui-ci, un mélange d'acétate de polyvinyle/povidone dans un rapport de 8:2 en tant que polymère hydrophobe qui confère une résistance à la rupture d'au moins 500N à ladite composition et de l'hydroxypropylméthylcellulose en tant que polymère hydrophile, dans lequel le polymère hydrophobe est à une concentration de 15 % p/p à 30 % p/p et le polymère hydrophile de 10 % p/p à 20 % p/p, dans lequel ledit procédé est extrusion thermofusible à une température inférieure à 180 C.

5. Procédé selon la 4^{ème} revendication, dans lequel tous les composants sont mélangés ensemble ou séparément dans une combinaison différente et le mélange résultant ou les mélanges résultants est/sont chauffé(s) dans l'extrudeuse au moins jusqu'au point de ramollissement du mélange acétate de polyvinyle/povidone et extrudé(s) à travers l'orifice de sortie de l'extrudeuse par application de force et l'extrudat encore plastique est cintré et façonné en une forme posologique, ou le produit extrudé cintré, refroidi et éventuellement réchauffé, est façonné en une forme posologique.

6. Procédé selon la 4^{ème} revendication qui comprend en outre un agent améliorant la viscosité, un solubilisant et un plastifiant.
